# EUROPÄISCHE PATENTANMELDUNG

(11) **EP 0 808 842 A2**
(43) Veröffentlichungstag der Anmeldung: **26.11.1997**
(21) Anmeldenummer: 97105575.1
(22) Anmeldetag: 04.04.1997
(51) Int. Cl.: C07H 15/04, B01F 17/56, A61K 7/075, C11D 1/04, C11D 1/66

(54) **Alkyl-2-acetamido-2-desoxy-gluco-pyranosid-uronsäuren und -Derivate, ihre Herstellung sowie ihre Verwendung als Tenside in kosmetischen und pharmazeutischen Zubereitungen**

(30) Priorität: 27.04.1996 DE 19617019
(71) Anmelder: Beiersdorf Aktiengesellschaft, 20245 Hamburg (DE)
(72) Erfinder: Schneider, Günther, Dr., 20253 Hamburg (DE); Schmidt-Lewerkühne, Hartmut, Dr., 22869 Schenefeld (DE); Thiem, Joachim, Prof.-Dr., 22391 Hamburg (DE); Scheel, Oliver, Dr., 22559 Hamburg (DE)

(57) **Zusammenfassung**

Alkyl-2-acetamido-2-desoxy-D-glucopyranosid-uronsäuren, bzw. deren pharmazeutisch oder kosmetisch akzeptablen Salze sowie ihre Verwendung als oberflächenaktive und/oder grenzflächenaktive Wirkstoffe, insbesondere als waschaktive Tenside oder Emulgatoren.

## Beschreibung

Die vorliegende Erfindung betrifft neue Wirkstoffe, deren Herstellung sowie deren Verwendung auf dem Gebiete der kosmetischen sowie der pharmazeutischen Dermatologie. Insbesondere betrifft die vorliegende Erfindung Wirkstoffe und kosmetische oder dermatologische Zubereitungen, solche Wirkstoffkombinationen enthaltend. Insbesondere betrifft die vorliegende Erfindung Wirkstoffe, welche Tensideigenschaften besitzen. Ferner betrifft die Erfindung kosmetische und dermatologische Zubereitungen mit einem Gehalt an solchen Substanzen. In einer bevorzugten Ausführungsform betrifft die vorliegende Erfindung kosmetische Reinigungsmittel.

Derartige Mittel sind an sich bekannt. Es handelt sich dabei im wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark. Das gebräuchlichste Tensid für kosmetische Reinigungsmittel ist das Natriumlaurylethersulfat. Obwohl es gute Waschkraft besitzt und - je nach Dosierung - recht gut haut- und schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Auch kosmetische Emulsionen enthalten grenzflächenaktive Stoffe, sogenannte Emulgatoren. Diese setzen die Grenzflächen zwischen den Phasen herab und bilden an der Phasengrenze Öl/Wasser Grenzflächenfilme aus. Dadurch wird dem irreversiblen Zusammenfließen der dispergierten Phase entgegengewirkt.

Leistungsfähige Emulgatoren zeichnen sich daher durch eine sehr gute Emulgier-, Solubilisierungs- sowie Dispergierungsfähigkelt aus. Es ist in hohem Maße wünschenswert, daß solche Substanzen keine Hautreizung auslösen. Die Auswahl solcher dem Stande der Technik bekannten Substanzen ist begrenzt. Es war daher eine Aufgabe der vorliegenden Erfindung, den Stand der Technik in dieser Hinsicht zu bereichern.

Vorteilhafte Reinigungsformulierungen, auch im Sinne der vorliegenden Erfindung, sind Duschpräparate.

Derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Im allgemeinen unterscheiden sich Präparate, welche für das Duschbad vorgesehen sind, nicht oder kaum von Wannenbadzubereitungen, abgesehen davon, daß bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Homschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Der Stand der Technik kennt Ölbadzubereitungen verschiedener Art, wobei die Eigenschaften der Fett- oder Ölphase durch Zugabe von oberflächenaktiven Substanzen variiert werden kann. Dabei können je nach Art und Menge der gewählten Bestandteile Zubereitungen formuliert werden, die auf der Badewasseroberfläche entweder spreitende Ölfilme, Öl-in-Wasser-Systeme oder auch Totalsolubilisate ergeben. Schäumende, aber auch wenig schäumende oder nicht schäumende Formulierungen sind möglich. Im allgemeinen beschränkt sich die Funktionalität derartiger Zubereitungen bei Ölbad- oder Ölcrèmebadzubereitungen auf die Rückfettung oder Überfettung der obersten Hautschichten.

Dennoch können pflegende Öle im Einzelfalle die schädigende Wirkung eines wenig hautfreundlichen Tensides bestenfalls überdecken, weswegen ein Ölbad oder ein Ölcrèmebad nicht besser sein kann als das oder die darin eingesetzten Tenside.

Es war somit die Aufgabe der vorliegenden Erfindung, den Mißständen des Standes der Technik in dieser Hinsicht abzuhelfen.

In einer weiteren bevorzugten Ausführungsform betrifft die vorliegende Erfindung kosmetische und dermatologische Zubereitungen zur Prophylaxe und Behandlung kosmetischer oder dermatologischer Zellveränderungen, insbesondere Hautveränderungen wie z.B. der Hautalterung, insbesondere der durch oxidative Prozesse hervorgerufenen Zellalterung, insbesondere der Hautalterung.

Schließlich war es Aufgabe der vorliegenden Erfindung, Herstellungsverfahren für solche Wirkstoffe zu konzipieren.

Es war überraschend und für den Fachmann nicht vorauszusehen, daß Alkyl-2-acetamido-2-desoxy-D-glucopyranosid-uronsäuren der allgemeinen Formel 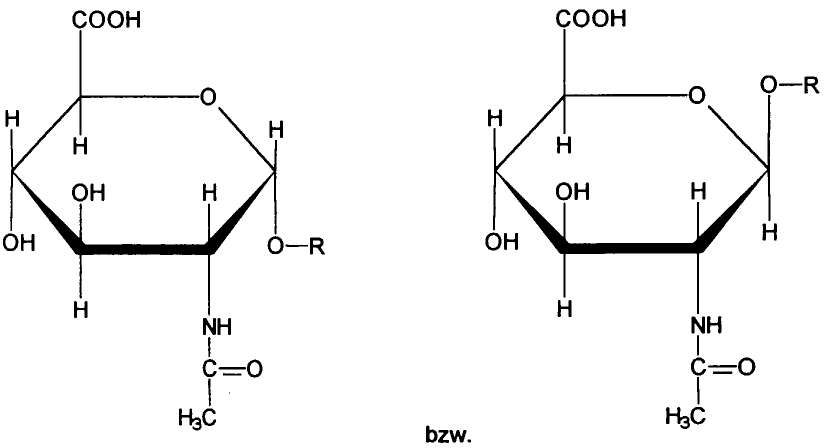 wobei R stellvertretend steht für die Gruppe bestehend aus verzweigtem und unverzweigtem Alkyl von 1 - 48 C-Atomen, insbesondere von 14 - 22, bevorzugt 16 - 18 C-Atomen, bzw. deren Salzen, sowie ein Verfahren zur Herstellung von Alkyl-2-acetamido-2-desoxy-D-glucopyranosid-uronsäuren der allgemeinen Formel 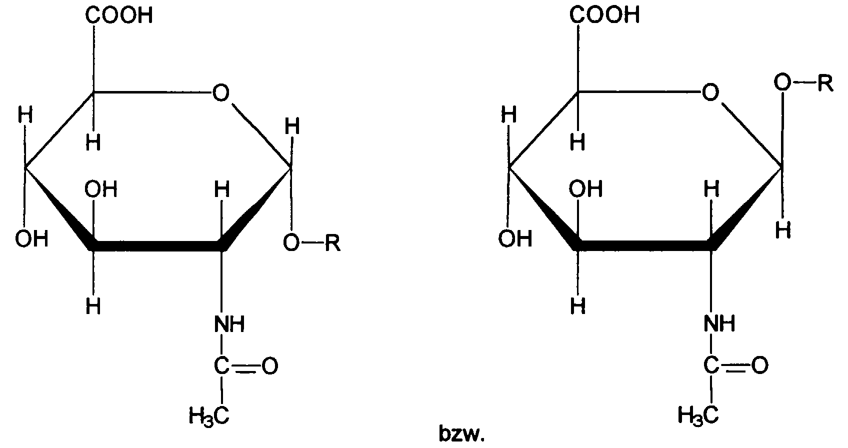 wobei R stellvertretend steht für die Gruppe bestehend aus verzweigtem und unverzweigtem Alkyl von 1 - 48 C-Atomen, insbesondere von 14 - 22, bevorzugt 16 - 18 C-Atomen, bzw. deren Salzen, dadurch gekennzeichnet, daß
(1) Chitin gemahlen wird
(2) das gemahlene Chitin mit Säure und einem ersten aliphatischen Alkohol niedriger Kettenlänge, bevorzugt einer Kettenlänge kleiner als C₈ versetzt wird, worauf
(3) gegebenenfalls das so erhaltene Reaktionsprodukt durch Dialyse, bevorzugt Elektrodialyse, gereinigt wird,
(4) ein zweiter, nicht mit dem ersten aliphatischen Alkohol identischer Alkohol, bevorzugt einer Kettenlänge von C₈ und länger, unter Säurekatalyse mit dem Reaktionsprodukt umgesetzt wird, wodurch der erste aliphatische Alkohol wieder frei und
   (4a) gegebenenfalls auf destillativem Wege entfernt wird,
(5) das Reaktionsprodukt der Oxidation unterworfen wird,
und die aus diesem Verfahren erhaltenen Alkyl-2-acetamid-2-desoxy-D-glucopyranosid-uronsäuren bzw. deren Salze sowie die neuen Zwischenprodukte in diesem Verfahren alle geschilderten Nachteile des Standes der Technik beseitigen.

Erfindungsgemäß ist folglich auch die Verwendung Alkyl-2-acetamido-2-desoxy-D-glucopyranosid-uronsäuren der allgemeinen Formel 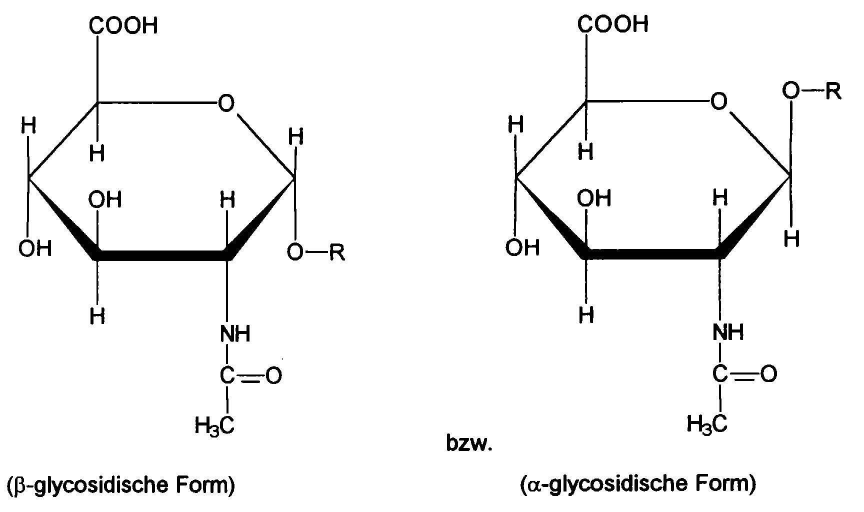 wobei R stellvertretend steht für die Gruppe bestehend aus verzweigtem und unverzweigtem Alkyl von 1 - 48 C-Atomen, insbesondere von 14 - 22, bevorzugt 16 - 18 C-Atomen, als oberflächenaktive und/oder grenzflächenaktive Wirkstoffe, insbesondere als waschaktive Tenside oder Emulgatoren, und wobei die Uronsäuren bevorzugt in Form ihrer Salze, insbesondere als Alkalisalze, davon bevorzugt das Natriumsalz, als Ammonium-, Alkylammonium-, Dialkylammonium-, Trialkylammonium- oder Tetraalkylammoniumsalze, vorliegen können.

Die erfindungsgemäß erhaltenen Substanzen zeichnen sich durch hervorragende Hautfreundlichkeit, sehr gute ober- und grenzflächenaktive Wirksamkeit und Verarbeitbarkeit in kosmetische und dermatologische Zubereitungen aus.

Auch Gemische aus α- und β-Glycosiden können erfindungsgemäß vorteilhaft sein. Jedenfalls sind dem Fachmann eine Fülle von Verfahren bekannt, welche die Anomeren zu trennen imstande sind, beispielsweise chromatographische Verfahren.

Die erfindungsgemäßen Alkyl-2-acetamido-2-desoxy-D-glucopyranosid-uronsäuren und deren vorteilhaften Salze leiten sich formal wie auch praktisch vom Chitin ab, welches durch die Strukturformel 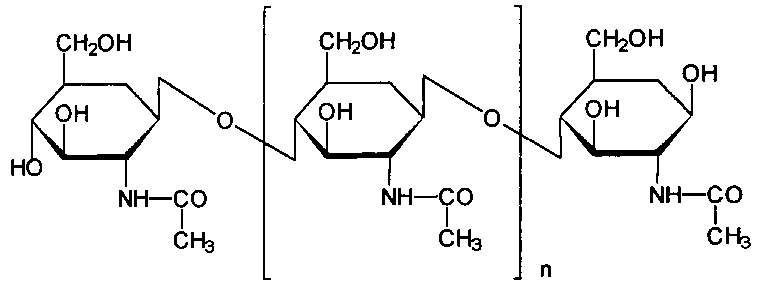 gekennzeichnet ist. Chitin ist wesentlicher Bestandteil des Ektoskeletts [ 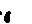 ο χ 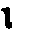 των = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Die Verwendung von Chitinderivaten, namentlich von Chitosan, einem Hydrolyseprodukt des Chitins, in kosmetischen Zubereitungen ist an sich bekannt. Chitosan, z.B., eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, 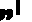 Lexikon der Hilfsstoffe für Pharmazie, Kosmetik und angrenzende Gebiete 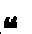 , vierte Auflage 1989, Editio Cantor, Aulendorf, S. 349, Stichwort 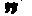 Chitosan 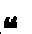 .

Auch die Verwendung anderer Zuckerderivate als oberflächenaktive Substanzen in Kosmetika, Dermatika und anderen Zubereitungen ist durchaus bekannt, beispielsweise aus den Patentanmeldungsschriften WO 93/07249, DE-OS 41 29 124, DE-OS 41 02 502, DE-OS 40 26 471 und DE OS 40 26 809.

Gleichwohl konnte der Stand der Technik keinen Hinweis auf die vorliegende Erfindung geben.

Erfindungsgemäß vorteilhaft wird ein Verfahren angewandt, welches sich am folgenden Reaktionsschema orientiert:

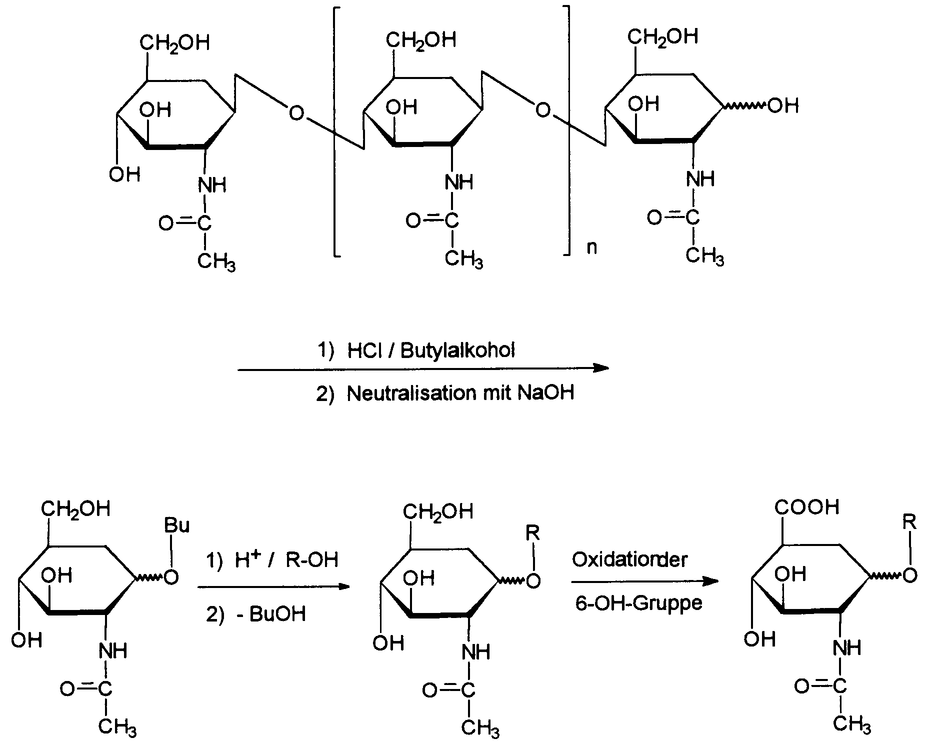

Besonders vorteilhafte Reaktionsbedingungen sehen vor, daß
(2) das gemahlene Chitin in der Wärme, vorteilhaft bei Temperaturen von 40 - 70° C, insbesondere ca 55° C mit Säure und einem ersten aliphatischen Alkohol versetzt wird.
Besonders vorteilhafte Reaktionsbedingungen sehen vor, daß
(4) ein zweiter, nicht mit dem ersten aliphatischen Alkohol identischer Alkohol, bevorzugt einer Kettenlänge von C₈ und länger, unter Säurekatalyse mit dem Reaktionsprodukt umgesetzt wird, wodurch der erste aliphatische Alkohol wieder frei und
   (4a) im Vakuum auf destillativem Wege entfernt wird.

Ein geeigneter Temperaturbereich für diesen Reaktionsschritt und für die destillative Entfernung des ersten aliphatischen Alkohols ist zwischen 80 und 100° C, bevorzugt ca. 90°C.
Die Säurekatalyse kann über saure Ionenaustauscherharze, z.B. Amberlite IR 120, mittels Camphersulfonsäure oder Salzsäure vorgenommen werden.
Am Ende dieses Reaktionsschrittes kann vorteilhaft eine Neutralisation, z.B. mit Natronlauge oder über basische Ionenaustauscherharze vorgenommen werden.

Weiterhin ist von Vorteil,
(5) das Reaktionsprodukt der Oxidation mit Pt/O₂ (Adamskatalysator) zu unterwerfen.

Erfindungsgemäß ist ein Anomerengemisch erhältlich, welches nach den dem Fachmanne geläufigen Methoden in die Anomeren aufgetrennt werden kann. Vorwiegend wird erfindungsgemäß das α-Anomere erhalten, für gewöhnlich in neunfachem Überschuß gegenüber dem β-Anomeren.

Es kann auch von Vorteil sein, von käuflichen Alkyl-2-acetamido-2-desoxy-glucopyranosiden auszugehen und lediglich den Reaktionsschritt (5) durchzuführen gemäß dem Reaktionsschema

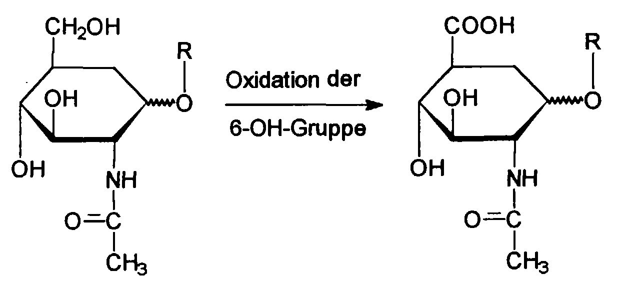

Ferner hat sich herausgestellt, daß auch die an sich bekannten Alkyl-2-acetamido-2-desoxy-glucopyranoside sich hervorragend als ober- bzw. grenzflächen aktive Substanzen eignen. Erfindungsgemäß ist daher auch die Verwendung von Alkyl-2-acetamido-2-desoxy-glucopyranosiden der allgemeinen Strukturformel 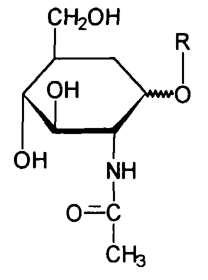 wobei R stellvertretend steht für die Gruppe bestehend aus verzweigtem und unverzweigtem Alkyl von 1 - 48 C-Atomen, insbesondere von 14 - 22, bevorzugt 16 - 18 C-Atomen, als oberflächenaktive und/oder grenzflächenaktive Wirkstoffe, insbesondere als waschaktive Tenside oder Emulgatoren.

Die erfindungsgemäßen kosmetischen oder dermatologischen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, insbesondere aber 0,01 Gew.-% bis 6 Gew.-%, bezogen auf das Gesamtgewicht des Mittels, an den erfindungsgemäßen Alkyl-2-acetamido-2-desoxy-D-glucopyranosid-uronsäuren.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vorn Typ Wasser-in-Öl-in-Wasser (W/O/W), ein Gel, einen festen Stift, eine Salbe oder auch ein Aerosol darstellen.

Es ist auch möglich und vorteilhaft im Sinne der vorliegenden Erfindung, die erfindungsgemäßen Alkyl-2-acetamido-2-desoxy-D-glucopyranosid-uronsäuren in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die erfindungsgemäßen Alkyl-2-acetamido-2-desoxy-D-glucopyranosid-uronsäuren auch mit Antioxidantien kombiniert werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann dabei vorteilhaft gewählt werden aus folgender Substanzgruppe:
- natürliche, synthetische und/oder partialsynthetische Öle, wie Triglyceride der Caprin- oder der Caprylsäure, vorzugsweise aber Rizinusöl;
- Fette, Wachse und andere natürliche synthetische und/oder partialsynthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- gesättigte Verbindungen wie Kohlenwasserstoffe natürlichen oder synthetischen Ursprungs (Vaseline, Squalan)

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) - 1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Alkyl-2-acetamido-2-desoxy-D-glucopyranosid-uronsäuren verwendet werden können, soll selbstverständlich nicht limitierend sein.

Gegenstand der Erfindung ist auch die Verwendung einer Kombination der erfindungsgemäßen Alkyl-2-acetamid-2-desoxy-D-glucopyranosid-uronsäuren mit mindestens einem UVB-Filter als Antioxidans bzw. die Verwendung einer Kombination der erfindungsgemäßen Tocopheryglycoside mit mindestens einem UVB-Filter als Antioxidans in einer kosmetischen oder dermatologischen Zubereitung.

Es kann auch von Vorteil sein, erfindungsgemäße Alkyl-2-acetamido-2-desoxy-D-glucopyranosid-uronsäuren mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination der erfindungsgemäßen Alkyl-2-acetamido-2-desoxy-D-glucopyranosid-uronsäuren mit mindestens einem UVA-Filter als Antioxidans bzw. die Verwendung einer Kombination der erfindungsgemäßen Tocopheryglycoside mit mindestens einem UVA-Filter als Antioxidans in einer kosmetischen oder dermatologischen Zubereitung.

Gegenstand der Erfindung ist auch die Verwendung einer Kombination der erfindungsgemäßen Alkyl-2-acetamido-2-desoxy-D-glucopyranosid-uronsäuren mit mindestens einem UVA-Filter und mindestens einem UVB-Filter als Antioxidans bzw. die Verwendung einer Kombination der erfindungsgemäßen Tocopheryglycoside mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäßen Alkyl-2-acetamido-2-desoxy-D-glucopyranosid-uronsäuren können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewährt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine weitere anionische, nicht-ionische oder amphotere und Betaine oberflächenaktive Substanz bzw. oberflächenaktive Betaine, oder auch Gemische aus solchen Substanzen, ferner erfindungsgemäße Alkyl-2-acetamido-2-desoxy-D-glucopyranosid-uronsäuren im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aërosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäße Wirkstoffkombinatonen in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haut und der Haare, die erfindungsgemäße Alkyl-2-acetamido-2-desoxy-D-glucopyranosid-uronsäuren enthalten, können als Emulsionen vorliegen, die vom nicht-ionischen oder kationischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an erfindungsgemäßen Alkyl-2-acetamido-2-desoxy-D-glucopyranosid-uronsäuren bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Alkyl-2-acetamido-2-desoxy-D-glucopyranosid-uronsäuren in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide und/oder Polyglycolether-Derivate.

Kosmetische Zubereitungen, die kosmetische Reinigungszubereitungen für die Haut darstellen, können in flüssiger oder fester Form vorliegen. Sie können neben den erfindungsgemäß verwendeten Alkyl-2-acetamido-2-desoxy-D-glucopyranosid-uronsäuren bzw. deren Salzen vorzugsweise mindestens eine anionische, nicht-ionische oder amphotere oberflächenaktive Substanz oder Gemische daraus enthalten, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäß verwendeten Alkyl-2-acetamido-2-desoxy-D-glucopyranosid-uronsäuren bzw. deren Salzen vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, antimikrobielle Stoffe, nückfettende Agentien, Komplexierungs- und Sequestrierungsagentien, Perlglanzagentien, Pflanzenextrakte, Vitamine, Wirkstoffe und dergleichen.

Vorzugsweise beträgt die Menge an erfindungsgemäß verwendeten Alkyl-2-acetamido-2-desoxy-D-glucopyranosid-uronsäuren in diesen Zubereitungen 0,001 Gew.-% bis 10 Gew.-%, insbesondere 0,1 Gew.-% bis 3 Gew.-%, bezogen auf das Gesamtgewicht der Zubereitungen.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäßen Alkyl-2-acetamido-2-desoxy-D-glucopyranosid-uronsäuren in kosmetische und dermatologische Formulierungen einarbeitet.

Die nachfolgenden Beispiele sollen die vorliegende Erfindung verdeutlichen, ohne sie einzuschränken.

### Herstellungsbeispiel 1

### Stearyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Stearylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 2

### Cetyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Cetylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 3

### Decyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Decylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 4

### Dodecyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Dodecylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 5

### Tetradecyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Tetradecylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 6

### Palmityl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Palmitylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Herstellungsbeispiel 7

### Eikosyl-2-acetamido-2-desoxy-D-glucopyranosid-uronsäure Na-salz

Gemahlenes Chitin wird bei 55° C mit Salzsäure und Butylalkohol versetzt und das so erhaltene Reaktionsprodukt durch Elektrodialyse gereinigt. Das Reaktionsprodukt wird mit Eikosylalkohol unter Säurekatalyse mit Camphersulfonsäure bei 90° C umgesetzt und der Butylalkohol unter Wasserstrahlvakuum bei 90° C abgezogen. das Produkt wird mit NaOH neutralisiert und das Produkt bei 60° C mit Adamskatalysator zum Endprodukt oxidiert.

### Beispiel 1

| (O/W-Emulsion): | |
|---|---|
| | Gew.-% |
| Cetyl- 2-acetamido-2-desoxy-D-glucopyranosid-uronsäure Na-salz | 0.75 |
| Sorbitanstearat | 2,00 |
| Vaseline | 1,0 |
| Paraffinöl, subliquidum | 11,00 |
| Octyldodecanol | 4,00 |
| Hydrierte Kokosfettsäureglyceride | 1,00 |
| Carbomer 2984 | 0,15 |
| Glycerin | 3,00 |
| Sorbit | 1,00 |
| Panthenol | 1,00 |
| Tocopherylacetat | 1,25 |
| Bisabolol | 0,10 |
| Parfüm, Konservierungsmittel, Farbstoffe, Antioxidantien | q.s. |
| Wasser | ad 100,00 |

### Beispiel 2

| (W/O-Emulsion): | |
|---|---|
| | Gew.-% |
| Stearyl-2-acetamido-2-desoxy-D-glucopyranosid-uronsäure Na-salz | 0,15 |
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Glycerin | 3,00 |
| Sorbit | 1,00 |
| Panthenol | 1,00 |
| Tocopherylacetat | 1,25 |
| Bisabolol | 0,10 |
| Magnesiumsulfat 7 H₂O | 0,70 |
| Parfüm, Konservierungsmittel, Farb stoffe, Antioxidantien | q.s |
| Wasser | ad 100,00 |

### Beispiel 3

| (Hydrodispersionsgel): | |
|---|---|
| | Gew.-% |
| Cetyl-2-acetamid-2-desoxy-D-glucopyranosid-uronsäure Na-salz | 0,25 |
| PEG-8 (Polythylenglycol 400) | 5,00 |
| Ethanol | 2,00 |
| Carbomer 2984 | 0,70 |
| Triglycerid, flüssig | 1,50 |
| Glycerin | 4,00 |
| Sorbit | 1,00 |
| Panthenol | 0,50 |
| Tocopherolacetat | 0.50 |
| Parfüm, Konservierungsmittel, Farb stoffe, Antioxidantien | q.s |
| Wasser | ad 100,00 |

### Beispiel 4

| (O/W-Emulsion): | |
|---|---|
| | Gew.-% |
| Cetyl-2-acetamido-2-desoxy-D-glucopyranosid-uronsäure Na-salz | 2,00 |
| Glycerinmonostearat | 2,00 |
| Vaseline | 1,00 |
| Paraffinöl, subliquidum | 11,00 |
| Octyldodecanol | 4,00 |
| Hydrierte Kokosfettsäureglyceride | 1,00 |
| Carbomer 2984 | 0,15 |
| Glycerin | 3,00 |
| Sorbit | 1,00 |
| Panthenol | 1,00 |
| Tocopherylacetat | 1,25 |
| Bisabolol | 0,10 |
| Parfüm, Konservierungsmittel, Farb stoffe, Antioxidantien | q.s |
| Wasser | ad 100,00 |

### Beispiel 5

| (W/O-Emulsion): | |
|---|---|
| | Gew.-% |
| Stearyl-2-Acetamido-2-desoxy-D-Glucopyranosid | 0.25 |
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Glycerin | 3,00 |
| Sorbit | 1,00 |
| Panthenol | 1,00 |
| Tocopherolacetat | 1,25 |
| Bisabolol | 0,10 |
| Magnesiumsulfat 7 H₂O | 0,70 |
| Parfüm, Konservierungsmittel, Farb stoffe, Antioxidantien | q.s |
| Wasser | ad 100,00 |

### Beispiel 6

| (Hydrodispersionsgel): | |
|---|---|
| | Gew.-% |
| Cetyl-2-acetamido-2-desoxy-D-glucopyranosid | 0,15 |
| PEG-8 (Polythylenglycol 400) | 5,00 |
| Ethanol | 2,00 |
| Carbomer 2984 | 0,70 |
| Triglycerid, flüssig | 1,50 |
| Glycerin | 4,00 |
| Sorbit | 1,00 |
| Panthenol | 0,50 |
| Tocopherolacetat | 0.50 |
| Parfüm, Konservierungsmittel, Farb stoffe, Antioxidantien | q.s |
| Wasser | ad 100,00 |

### Beispiel 7

| Shampoo | |
|---|---|
| | Gew.-% |
| Cetyl-2-acetamido-2-desoxy-D-glucopyranosid-uronsäure Na-salz | 5,00 |
| Natrium Laurylethersulfat ( 27,5 %) | 25,00 |
| Natriumchlorid | q.s. |
| Perlglanzmittel | q.s. |
| Parfüm, Konservierungsmittel, Farb stoffe, Antioxidantien | q.s |
| Wasser | ad 100,00 |

### Beispiel 8

| Shampoo | |
|---|---|
| | Gew.-% |
| Stearyl-2-acetamido-2-desoxy-D-glucopyranosid-uronsäure Na-salz | 4,00 |
| Alkylpolyglucose ( 50 %) | 25,00 |
| Natriumchlorid | q.s. |
| Perlglanzmittel | q.s. |
| Parfüm, Konservierungsmittel, Farb stoffe, Antioxidantien | q.s |
| Wasser | ad 100,00 |

### Beispiel 9

| Shampoo | |
|---|---|
| | Gew.-% |
| Stearyl-2-acetamido-2-desoxy-D-glucopyranosid-uronsäure Na-salz | 5,00 |
| Natrium Laurylethersulfat ( 27,5 %) | 14,00 |
| Alkylpolyglucose ( 50 %) | 8,00 |
| Natriumchlorid | q.s. |
| Perlglanzmittel | q.s. |
| Parfüm, Konservierungsmittel, Farb stoffe, Antioxidantien | q.s |
| Wasser | ad 100,00 |

### Beispiel 9

| Gesichtsreinigungs-Waschlotion | |
|---|---|
| | Gew.-% |
| Stearyl-2-acetamido-2-desoxy-D-glucopyranosid-uronsäure Na-salz | 5,00 |
| Natrium cocoamphoacetate (30%) | 10,00 |
| Parfüm, Konservierungsmittel, Verdickungsmittel Farb stoffe, Antioxidantien | q.s |
| Wasser | ad 100,00 |

### Beispiel 10

| Duschbad klar | |
|---|---|
| | Gew.-% |
| Palmityl-2-acetamido-2-desoxy-D-glucopyranosid-uronsäure Na-salz | 5,00 |
| Natrium Laurylethersulfat ( 27,5 %) | 25,00 |
| Alkylpolyglucose ( 50 %) | 4,00 |
| Perlglanzmittel | q.s. |
| Natriumchlorid | q.s. |
| Parfüm, Konservierungsmittel, Verdickungsmittel Farb stoffe, Antioxidantien | q.s. |
| Wasser | ad 100,00 |

## Patentansprüche

1. Alkyl-2-acetamido-2-desoxy-D-glucopyranosid-uronsäuren der allgemeinen Formel 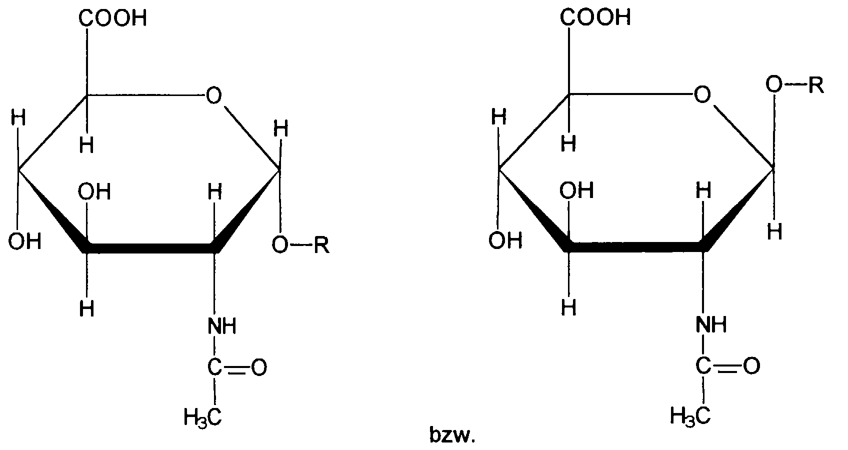 wobei R stellvertretend steht für die Gruppe bestehend aus verzweigtem und unverzweigtem Alkyl von 1 - 48 C-Atomen, verzweigtem und unverzweigtem Acyl von 1 - 48 C-Atomen, bzw. deren pharmazeutisch oder kosmetisch akzeptablen Salze.

2. Verfahren zur Herstellung von Alkyl-2-acetamido-2-desoxy-D-glucopyranosid-uronsäuren nach Anspruch 1, dadurch gekennzeichnet, daß
(1) Chitin gemahlen wird
(2) das gemahlene Chitin mit Säure und einem ersten aliphatischen Alkohol versetzt wird, worauf
(3) gegebenenfalls das so erhaltene Reaktionsprodukt durch Dialyse, vorzugsweise Elektrodialyse, gereinigt wird,
(4) ein zweiter, nicht mit dem ersten aliphatischen Alkohol identischer Alkohol unter Säurekatalyse mit dem Reaktionsprodukt umgesetzt wird, wodurch der erste aliphatische Alkohol wieder frei und
(4a) gegebenenfalls auf destillativem Wege entfernt wird,
(5) das Reaktionsprodukt der Oxidation unterworfen wird.

3. Verwendung von Alkyl-2-acetamido-2-desoxy-D-glucopyranosid-uronsäuren nach Anspruch 1 als oberflächenaktive und/oder grenzflächenaktive Wirkstoffe, insbesondere als waschaktive Tenside oder Emulgatoren, und wobei die Uronsäuren auch in Form ihrer Salze insbesondere als Alkalisalze, davon bevorzugt das Natriumsalz, als Ammonium-, Alkylammonium-, Dialkylammonium-, Trialkylammonium- oder Tetraalkylammoniumsalze, vorliegen können.

4. Verwendung von Alkyl-2-acetamido-2-desoxy-glucopyranosiden der allgemeinen Strukturformel 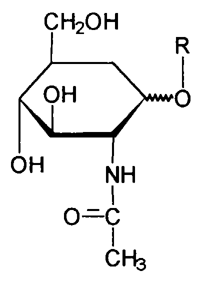 wobei R stellvertretend steht für die Gruppe bestehend aus verzweigtem und unverzweigtem Alkyl von 1 - 48 C-Atomen, insbesondere von 14 - 22, bevorzugt 16 - 18 C-Atomen, als oberflächenaktive und/oder grenzflächenaktive Wirkstoffe, insbesondere als waschaktive Tenside oder Emulgatoren.
